# FASCICULE DE BREVET EUROPEEN

(11) **EP 0 786 249 B1**
(45) Date de publication et mention de la délivrance du brevet: **15.05.2002**
(21) Numéro de dépôt: 96402888.0
(22) Date de dépôt: 24.12.1996
(51) Int. Cl.: A61K 7/48

(54) **Composition gélifiée stable contenant un électrolyte, un agent actif et de la cétylhydroxyéthylcellulose**
Stabile Gelzusammensetzung enthaltend ein Electrolyt, eine Aktivsubstanz und Cetylhydroxyethylcellulose
Stable gel composition containing an electrolyte, an active agent and cetyl hydroxyethylcellulose

(30) Priorité: 23.01.1996 FR 9600742
(43) Date de publication de la demande: 30.07.1997
(73) Titulaire: L'OREAL, 75008 Paris (FR)
(72) Inventeur: Cantin, Hervé, 91420 Morangis (FR); Gagnebien, Didier, 92320 Chatillon (FR)
(74) Mandataire: Lhoste, Catherine

(56) Documents cités:
- EP-A- 0 654 270
- DE-A- 3 421 443
- US-A- 4 618 491
- SEIFEN, OLE, FETTE, WACHSE JOURNAL, vol. 120, no. 15, 1994, AUGSBURG, DE, pages 944-948, XP000483287 J. J. DE BRUIN: "Hydrophobically modified cellulose ethes foe personal care"

## Description

La présente invention se rapporte à une composition topique gélifiée stable, comprenant au moins un actif cosmétique et/ou dermatologique et pouvant contenir une quantité importante d'électrolyte, et à son utilisation notamment pour le traitement et le soin de la peau humaine, du cuir chevelu, des muqueuses, des ongles et des cheveux.

Dans les domaines cosmétique, dermatologique et pharmaceutique, il est connu d'utiliser des compositions topiques sous forme de gels aqueux ou d'émulsions comportant des gélifiants qui donnent de la consistance à ces compositions. La majorité des gélifiants utilisés classiquement sont des polymères carboxyvinyliques, que l'on neutralise par une base.

Il arrive cependant que certains composés que l'on souhaite utiliser dans ces compositions ne permettent pas l'emploi des gélifiants cités précédemment par suite d'incompatibilité.

On sait par exemple que les électrolytes (sels inorganiques et organiques) sont incompatibles avec les polymères carboxyvinyliques neutralisés du fait qu'ils "cassent" l'émulsion et la liquéfient. Ainsi, des compositions contenant des polymères carboxyvinyliques et des électrolytes sont dépourvues de consistance, ce qui va à l'encontre du résultat recherché par l'emploi d'un gélifiant.

Or, il peut être souhaitable d'introduire des électrolytes dans des compositions épaissies, notamment topiques, et parfois même en une quantité relativement importante, notamment quand ces électrolytes ont un effet bénéfique sur la peau ou les cheveux et sont associés à des actifs cosmétiques et/ou dermatologiques ayant eux-mêmes des effets bénéfiques pour la peau, en particulier lorsque ces actifs ont un effet secondaire irritant. Le gélifiant doit être compatible avec les électrolytes tout en conservant l'efficacité de l'actif et des électrolytes. En outre, la composition doit être limpide et stable.

Une solution consiste à utiliser, à la place des polymères carboxyvinyliques, des gélifiants de type polysaccharidique tels que les gommes guar ou xanthane ou les dérivés cellulosiques. Ainsi, le document EP-A-654270 décrit une composition topique destinée au traitement de l'acné et des dermatites séborrhéiques, comportant un mélange de sels et, comme gélifiant, un dérivé de cellulose tel que l'hydroxyéthylcellulose.

Malheureusement, les compositions à base de dérivés de cellulose conformes à ce document, et notamment les gels aqueux ne comportant pas de phase grasse, n'ont pas une texture lisse et présentent au contraire un aspect grumeleux peu agréable à voir. En outre, ils laissent la peau « comme mouillée » après application, ces compositions ne pénétrant pas suffisamment dans la peau. Tout ceci rend leur utilisation rédhibitoire dans les domaines cosmétique et/ou dermatologique.

L'association de ces dérivés de cellulose avec un autre agent épaississant tel qu'un silicate, comme décrit par exemple dans le document WO-A-93/8230, donne des compositions où subsistent les mêmes inconvénients qu'indiqués ci-dessus (aspect grumeleux).

Il subsiste donc le besoin de compositions gélifiées contenant des électrolytes et ne présentant pas les inconvénients rencontrés avec les gélifiants connus, notamment d'inconsistance, d'instabilité, d'aspect grumeleux, de sensation désagréable au toucher et d'incompatibilité avec ces électrolytes.

La demanderesse a trouvé de façon inattendue un gélifiant permettant de stabiliser les compositions contenant une forte teneur en électrolyte.

Aussi, la présente invention a pour objet une composition topique gélifiée comportant une phase aqueuse comprenant au moins un actif cosmétique et/ou dermatologique, au moins un électrolyte, et comme gélifiant la cétylhydroxyéthylcellulose selon les revendications 1 ou 2.

La composition obtenue est limpide et a la viscosité souhaitée, c'est-à-dire supérieure à quelques poises (5 à 6 poises, équivalent à 0,5 à 0,6 Pa.s).

Certes, le document Seifen, Ole, Fette, Wachse Journal, vol.120, n°15, 1994 décrit l'utilisation d'hydroxyéthylcellulose hydrophobiquement modifiée dans des compositions de soin de la peau et la compatibilité d'un tel composé avec différents types de sels. Toutefois, ce document ne décrit pas l'association avec des actifs cosmétiques. En outre, il ne suggère pas que le gélifiant particulier utilisé selon la présente invention permette d'obtenir des compositions limpides et stables malgrè la présence d'actifs et d'une grande quantité de sels.

Par composition gélifiée, on entend aussi bien un gel aqueux ou hydroalcoolique, contenant ou non une huile, qu'une émulsion eau-dans-huile ou huile-dans-eau.

L'électrolyte peut être présent dans la composition notamment en une quantité allant de 0,5 à 40 % en poids, et de préférence de 1 à 20 % en poids par rapport au poids total de la composition.

Le gélifiant peut être utilisé en une quantité allant de 0,1 à 10 % en poids, et de préférence de 0,5 à 3 % en poids par rapport au poids total de la composition.

Le sel est choisi parmi le nitrate de calcium, de magnésium ou de strontium, le borate de calcium ou de magnésium, le chlorure de calcium, de sodium, de magnésium, de strontium, de néodyme ou de manganèse, le sulfate de magnésium ou de calcium, l'acétate de calcium ou de magnésium, et leurs mélanges.

L'actif peut être choisi notamment parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, antiseptiques, les actifs pour traiter les signes du vieillissement et/ou les agents modulant la différenciation et/ou la prolifération et/ou la pigmentation cutanée. Il peut s'agir aussi de vitamines telles que la vitamine C.

L'actif peut être en particulier choisi parmi les actifs à effet secondaire irritant tels que les α-hydroxy-acides, les β-hydroxy-acides, les α-céto-acides, les β-céto-acides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés.

Comme α-hydroxy-acides, on peut citer les acides citrique, malique, glycolique, tartrique, mandélique, lactique. Comme β-hydroxy-acides, on peut citer l'acide salicylique ainsi que ses dérivés acylés, les acides hydroxy-2 alcanoïques et leurs dérivés comme l'acide hydroxy-2-méthyl-3-benzoïque et l'acide hydroxy-2-méthoxy-3-benzoïque. Comme dérivé de l'acide salicylique, on peut citer notamment ceux décrits dans les documents FR-A-2581542 et EP-A-378936, et notamment les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique. On peut aussi utiliser ceux décrits dans le document EP-A-570230.

Il peut s'agir aussi d'un actif à effet secondaire irritant tels que les teintures ou colorants capillaires (paraphénylènediamine et ses dérivés, aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone), les pigments, les tensioactifs et les filtres chimiques.

Ces actifs peuvent selon la quantité utilisée et selon la sensibilité de la peau de l'utilisateur, se révéler plus ou moins irritants ou sensibilisants.

Par ailleurs, la quantité d'actif peut varier dans une large mesure en fonction de la nature et/ou de la fonction de l'actif. Cette quantité peut aller par exemple de 0,001 à 30 % du poids total de la composition.

La composition selon l'invention destinée à un soin ou un traitement topique. doit contenir un milieu topiquement acceptable, c'est-à-dire compatible avec la peau, les muqueuses, les ongles, le cuir chevelu et les cheveux. Elle peut se présenter sous toutes les formes galéniques appropriées pour une application topique, et notamment sous forme de gel aqueux ou hydroalcoolique, d'émulsion eau-dans-huile ou huile-dans-eau ou de dispersion aqueuse à base de vésicules lipidiques ioniques et/ou non-ioniques, contenant ou non une huile dispersée. Elle peut constituer par exemple un sérum, une crème ou un lait.

Les quantités des différents constituants des compositions selon l'invention sont celles classiquement utilisées dans le domaine considéré.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

De façon connue, la composition de l'invention peut contenir également des adjuvants habituels dans les domaines cosmétique et/ou dermatologique, tels que les agents hydratants (glycérine, sel de sodium de l'acide pyrrolidone carboxylique, D-panthénol), les conservateurs, les antioxydants, les agents complexants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme huiles utilisables dans l'invention, on peut citer les huiles minérales (isoparaffine hydrogénée), les huiles d'origine végétale (huile d'abricot), les huiles d'origine animale, les huiles de synthèse (tétraéthylhexanoate de pentaérythrityle), les huiles siliconées (cyclomethicone), et les huiles fluorées. On peut aussi utiliser comme matières grasses des alcools gras (alcool cétylique), des acides gras, des cires et composés cireux (diéthanolamine d'acide gras de coprah), et des gommes (gomme de silicone).

Comme émulsionnants utilisables dans l'invention, on peut citer par exemple le mélange de monostéarate de glycéryle et de stéarate de polyéthylèneglycol vendu par la société ICI sous la dénomination Arlacel 165, le tristéarate de sorbitan (Span 65 vendu par la société ICI), le stéarate de PEG-40 (Myrj 52 vendu par la société ICI) et le cetyl dimethicone copolyol (Abil EM-90 vendu par la société Goldschmidt).

La composition selon l'invention peut notamment être utilisée pour le traitement et le soin de la peau, des muqueuses, du cuir chevelu et/ou des cheveux, en particulier pour le traitement de la peau sensible et du cuir chevelu sensible et/ou pour hydrater la peau.

Aussi, la présente invention a encore pour objet l'utilisation de la composition telle que définie ci-dessus dans ou pour la fabrication d'une composition cosmétique, pharmaceutique et/ou dermatologique pour traiter la peau sensible et/ou le cuir chevelu sensible et/ou pour hydrater la peau. Pour de plus amples détails concernant la peau sensible, on peut se référer au document EP-A-680749.

La présente invention a aussi pour objet un procédé de traitement cosmétique et/ou dermatologique de la peau, du cuir chevelu, des cheveux, et/ou des muqueuses, caractérisé par le fait que l'on applique sur la peau, le cuir chevelu, les cheveux, et/ou les muqueuses, une composition telle que définie ci-dessus.

Les exemples ci-après de compositions selon l'invention, sont donnés à titre d'illustration. Les quantités y sont données en % en poids.

### Exemple 1 Gel hydratant

- Chlorure de strontium, 6 H₂O 6 %
- Acide glycolique 1 %
- Glycérine 5 %
- Cétylhydroxyéthylcellulose (Polysurf 67 vendu par la société Aqualon) 1 %
- Parfum 0,2 %
- Peptisant de parfum 1 %
- D-panthénol 1 %
- Sel de sodium de l'acide pyrrolidone carboxylique 1,5 %
- Conservateur 0,2 %
- Eau déminéralisée qsp 100 %

Le gel obtenu a un aspect lisse et est agréable à appliquer. Il est apte à hydrater la peau, notamment la peau sensible.

### Exemple 2 : Crème de jour antivieillisement

### Phase huileuse :

- Isoparaffine hydrogénée 10 %
- Cyclopentadiméthylsiloxane 10 %
- Alcool cétylique 1 %
- Acide n-octanoyl-5-salicylique 0,2 %

### Phase aqueuse :

- Nitrate de calcium tétrahydraté 6 %
- Cétylhydroxyéthylcellulose (Polysurf 67 vendu par la société Aqualon) 1,5 %
- Conservateur 0,2 %
- Eau déminéralisée qsp 100 %

Le mode opératoire pour préparer cette composition a consisté à mélanger les constituants de la phase aqueuse à chaud en agitant jusqu'à homogénéisation et à introduire, sous agitation, la phase huileuse préalablement homogénéisée dans la phase aqueuse obtenue.

La crème obtenue est une crème blanche, douce à l'application et apte à traiter la peau du visage, même très sensible.

### Exemple 3 Crème de jour hydratante

### Phase huileuse :

- Tétraéthylhexanoate de pentaérythrityle 0,45 %
- Alcool cétylique 0,075 %
- Diéthanolamine d'acide gras de coprah 0,075 %
- Arlacel 165 vendu par la société ICI 0,28 %

### Phase aqueuse :

- Chlorure de calcium anhydre 2,8 %
- Acide glycolique 0,5 %
- Cétylhydroxyéthylcellulose (Polysurf 67 vendu par la société Aqualon) 1 %
- Glycérine 3 %
- Eau déminéralisée qsp 100 %

Le mode opératoire pour préparer cette composition a consisté à préparer séparément les deux phases jusqu'à homogénéité et à introduire la phase huileuse dans la phase aqueuse sous agitation.

La crème obtenue est une crème blanche apte à hydrater la peau.

### Exemple 4 Crème de jour hydratante

On peut préparer une crème identique à celle de l'exemple 3 en mettant 10 % de chlorure de calcium au lieu des 2,8 %. On obtient une crème gélifiée stable, apte à hydrater la peau.

### Exemple 5 : Crème antivieillissement (émulsion huile-dans-eau)

### Phase huileuse :

- Tristéarate de sorbitan (Span 65 vendu par la société ICI) 0,9 %
- Stéarate de PEG-40 (Myrj 52 vendu par la société ICI) 2 %
- Alcool cétylique 4 %
- Isoparaffine hydrogénée 10 %
- Cyclométhicone (DC 245 vendu par la société Dow Corning) 10 %

### Phase aqueuse :

- Chlorure de strontium, 6 H₂O 6 %
- Acide lactique 5 %
- Cétylhydroxyéthylcellulose (Polysurf 67 vendu par la société Aqualon) 0,7 %
- Eau déminéralisée qsp 100 %

Le mode opératoire pour préparer cette composition a consisté à préparer séparément les deux phases jusqu'à homogénéité et à introduire la phase huileuse dans la phase aqueuse sous agitation.

On obtient une crème stable, apte à traiter la peau du visage, même très sensible.

### Exemple 6 : Crème antivieillissement (émulsion eau-dans-huile)

### Phase huileuse :

- Cetyl dimethicone copolyol (Abil EM-90 vendu par la société Goldschmidt) 3 %
- Cyclométhicone (DC 245 vendu par la société Dow Corning) 12 %
- Gomme de silicone (DC 1403 vendu par la société Dow Corning) 3 %
- Huile d'abricot 3 %

### Phase aqueuse :

- Chlorure de strontium, 6 H₂O 6 %
- Acide glycolique 5 %
- Cétylhydroxyéthylcellulose (Polysurf 67 vendu par la société Aqualon) 0,7 %
- Eau déminéralisée qsp 100 %

Le mode opératoire pour préparer cette composition a consisté à préparer séparément les deux phases jusqu'à homogénéité et à introduire la phase aqueuse dans la phase huileuse sous agitation.

On obtient une crème stable, apte à traiter la peau du visage, même très sensible.

## Revendications

1. Composition topique gélifiée comportant une phase aqueuse comprenant au moins un actif cosmétique et/ou dermatologique, au moins un électrolyte, et comme gélifiant la cétylhydroxyéthylcellulose, l'actif étant choisi parmi l'actif étant choisi parmi les agents kératolytiques, anti-radicaux libres, anti-séborrhéiques, antipelliculaires, les agents modulant la différenciation et/ou la prolifération et/ou la pigmentation cutanée, les vitamines, les α-hydroxy-acides, les β-hydroxy-acides, les α-céto-acides, les β-céto-acides, les rétinoïdes, les anthralines, les anthranoïdes, les peroxydes, la vitamine D et ses dérivés, les teintures ou colorants capillaires, les agents antitranspirants, les actifs dépilatoires ou de permanentes, les actifs dépigmentants, les pigments et les filtres chimiques, et l'électrolyte étant choisi parmi le nitrate de calcium, de magnésium ou de strontium, le borate de calcium ou de magnésium, le chlorure de calcium, de magnésium, de sodium, de strontium, de néodyme ou de manganèse, le sulfate de magnésium ou de calcium, l'acétate de calcium ou de magnésium, et leurs mélanges.

2. Composition topique gélifiée comportant une phase aqueuse comprenant au moins un actif dermatologique, au moins un électrolyte, et comme gélifiant la cétylhydroxyéthylcellulose, l'actif étant choisi parmi les agents antiparasitaires, antifongiques, antiviraux, anti-inflammatoires, antiprurigineux, anesthésiques, antiacnéiques, antiseptiques, le minoxidil, les antimétabolites, et l'électrolyte étant choisi parmi le nitrate de calcium, de magnésium ou de strontium, le borate de calcium ou de magnésium, le chlorure de calcium, de magnésium, de sodium, de strontium, de néodyme ou de manganèse, le sulfate de magnésium ou de calcium, l'acétate de calcium ou de magnésium, et leurs mélanges.

3. Composition selon la revendication 1 ou 2, **caractérisée en ce que** le gélifiant est présent en une concentration allant de 0,1 à 10 % en poids par rapport au poids total de la composition.

4. Composition selon l'une quelconque des revendications précédentes, **caractérisée en ce que** l'électrolyte est présent en une concentration allant de 0,5 à 40 % en poids par rapport au poids total de la composition.

5. Composition selon l'une quelconque des revendications 1, 3 et 4, **caractérisée en ce que** l'actif est choisi parmi l'acide citrique, l'acide malique, l'acide glycolique, l'acide tartrique, l'acide mandélique, l'acide lactique, l'acide salicylique et ses dérivés.

6. Composition selon l'une quelconque des revendications précédentes, **caractérisée en ce que** l'actif est une quantité allant de 0,001 à 30 % du poids total de la composition.

7. Composition selon l'une quelconque des revendications précédentes, **caractérisée en ce qu'**elle comprend, en outre, une phase grasse.

8. Utilisation cosmétique de la composition cosmétique selon l'une quelconque des revendications 1 et 3 à 7, pour hydrater la peau.

9. Utilisation de la composition selon l'une quelconque des revendications 2 à 4 et 6 à 7 pour la fabrication d'une composition pharmaceutique ou dermatologique pour traiter la peau sensible et/ou le cuir chevelu sensible.

10. Procédé de traitement cosmétique de la peau, du cuir chevelu, des cheveux, des ongles et/ou des muqueuses, **caractérisé par le fait que** l'on applique sur la peau, le cuir chevelu, les cheveux, les ongles et/ou les muqueuses, une composition selon l'une quelconque des revendications 1 et 3 à 7.

## Patentansprüche

1. Topische Zusammensetzung in Gelform, die eine wäßrige Phase aufweist und die mindestens einen kosmetischen und/oder dermatologischen Wirkstoff, mindestens einen Elektrolyten und als Gelbildner Cetylhydroxyethylcellulose enthält, wobei der Wirkstoff unter den Keratolytika, Radikalfängern für freie Radikale, Wirkstoffen gegen Seborrhoe, Antischuppenmitteln, Mitteln, die die Differenzierung und/oder Proliferation und/oder Pigmentierung der Haut modulieren, Vitaminen, a-Hydroxysäuren, β-Hydroxysäuren, α-Ketosäuren, β-Ketosäuren, Retinoiden, Anthralinen, Anthranoiden, Peroxiden, Vitamin D und seinen Derivaten, Haarfärbemitteln oder Farbstoffen für das Haar, Antitranspirantien, Depilationsmitteln, Mitteln für die permanente Verformung, depigmentierenden Wirkstoffen, Pigmenten und chemischen Filtern ausgewählt ist, und wobei der Elektrolyt ausgewählt ist unter Calciumnitrat, Magnesiumnitrat oder Strontiumnitrat, Calciumborat oder Magnesiumborat, Calciumchlorid, Magnesiumchlorid, Natriumchlorid, Strontiumchlorid, Neodymchlorid oder Manganchlorid, Magnesiumsulfat oder Calciumsulfat, Calciumacetat oder Magnesiumacetat und deren Gemischen.

2. Topische Zusammensetzung in Gelform, die eine wäßrige Phase aufweist und die mindestens einen dermatologischen Wirkstoff, mindestens einen Elektrolyten und als Gelbildner Cetylhydroxyethylcellulose enthält, wobei der Wirkstoff unter den antiparasitären Wirkstoffen, Antimykotika, antiviralen Wirkstoffen, entzündungshemmenden Wirkstoffen, juckreizstillenden Wirkstoffen, anästhesierenden Wirkstoffen, Wirkstoffen gegen Akne, antiseptischen Wirkstoffen, Minoxidil und Antimetaboliten ausgewählt ist, und wobei der Elektrolyt ausgewählt ist unter Calciumnitrat, Magnesiumnitrat oder Strontiumnitrat, Calciumborat oder Magnesiumborat, Calciumchlorid, Magnesiumchlorid, Natriumchlorid, Strontiumchlorid, Neodymchlorid oder Manganchlorid, Magnesiumsulfat oder Calciumsulfat, Calciumacetat oder Magnesiumacetat und deren Gemischen.

3. Zusammensetzung nach Anspruch 1 oder 2, **dadurch gekennzeichnet, daß** der Gelbildner in einer Menge von 0,1 bis 10 Gew.-%, bezogen auf das Gesamtgewicht der Zusammensetzung, vorliegt.

4. Zusammensetzung nach einem der vorhergehenden Ansprüche, **dadurch gekennzeichnet, daß** der Elektrolyt in einer Konzentration im Bereich von 0,5 bis 40 Gew.-%, bezogen auf das Gesamtgewicht der Zusammensetzung, vorliegt.

5. Zusammensetzung nach einem der Ansprüche1, 3 und 4, **dadurch gekennzeichnet, daß** der Wirkstoff unter Citronensäure, Äpfelsäure, Glykolsäure, Weinsäure, Mandelsäure, Milchsäure, Salicylsäure und ihren Derivaten ausgewählt ist.

6. Zusammensetzung nach einem der vorhergehenden Ansprüche, **dadurch gekennzeichnet, daß** der Wirkstoff in einer Menge von 0,001 bis 30 % des Gesamtgewichts der Zusammensetzung vorliegt.

7. Zusammensetzung nach einem der vorhergehenden Ansprüche, **dadurch gekennzeichnet, daß** sie ferner eine Fettphase enthält.

8. Kosmetische Verwendung der kosmetischen Zusammensetzung nach einem der Ansprüche 1 und 3 bis 7 zur Hydratisierung der Haut.

9. Verwendung der Zusammensetzung nach einem der Ansprüche 2 bis 4 und 6 bis 7 zur Herstellung einer pharmazeutischen oder dermatologischen Zusammensetzung zur Behandlung von empfindlicher Haut und/oder empfindlicher Kopfhaut.

10. Verfahren zur kosmetischen Behandlung der Haut, der Kopfhaut, der Haare, der Nägel und/oder der Schleimhäute, **dadurch gekennzeichnet, daß** auf die Haut, die Kopfhaut, die Haare, die Nägel und/oder die Schleimhäute eine Zusammensetzung nach einem der Ansprüche 1 und 3 bis 7 aufgetragen wird.

## Claims

1. Topical gel composition containing an aqueous phase comprising at least one cosmetic and/or dermatological active agent, at least one electrolyte, and, as gelling agent, cetyl hydroxyethyl cellulose, the active agent being chosen from keratolytic, anti-free radical, antiseborrhoeic and antidandruff agents, agents modulating skin differentiation and/or proliferation and/or pigmentation, vitamins, α-hydroxy acids, β-hydroxy acids, α-keto acids, β-keto acids, retinoids, anthralins, anthranoids, peroxides, vitamin D and its derivatives, hair dyes or colorants, anti-perspirants, depilatory or permanent-waving active agents, depigmenting active agents, pigments and chemical screening agents, and the electrolyte being chosen from calcium, magnesium or strontium nitrate, calcium or magnesium borate, calcium, magnesium, sodium, strontium, neodymium or manganese chloride, magnesium or calcium sulphate, calcium or magnesium acetate, and mixtures thereof.

2. Topical gel composition containing an aqueous phase comprising at least one dermatological active agent, at least one electrolyte, and, as gelling agent, cetyl hydroxyethyl cellulose, the active agent being chosen from antiparasitic, antifungal, antiviral, antiinflammatory, antipruritic, anaesthetic, anti-acne and antiseptic agents, minoxidil and antimetabolites, and the electrolyte being chosen from calcium, magnesium or strontium nitrate, calcium or magnesium borate, calcium, magnesium, sodium, strontium, neodymium or manganese chloride, magnesium or calcium sulphate, calcium or magnesium acetate, and mixtures thereof.

3. Composition according to Claim 1 or 2, **characterized in that** the gelling agent is present in a concentration ranging from 0.1 to 10% by weight relative to the total weight of the composition.

4. Composition according to any one of the preceding claims, **characterized in that** the electrolyte is present in a concentration ranging from 0.5 to 40% by weight relative to the total weight of the composition.

5. Composition according to any one of Claims 1, 3 and 4, **characterized in that** the active agent is chosen from citric acid, malic acid, glycolic acid, tartaric acid, mandelic acid, lactic acid, salicylic acid and its derivatives.

6. Composition according to any one of the preceding claims, **characterized in that** the active agent is present in a quantity ranging from 0.001 to 30% of the total weight of the composition.

7. Composition according to any one of the preceding claims, **characterized in that** it comprises, in addition, a fatty phase.

8. Cosmetic use of the cosmetic composition according to any one of Claims 1 and 3 to 7, for moisturizing the skin.

9. Use of the composition according to any one of Claims 2 to 4 and 6 to 7, for the manufacture of a pharmaceutical or dermatological composition for treating sensitive skin and/or a sensitive scalp.

10. Method for the cosmetic treatment of the skin, the scalp, the hair, the nails and/or the mucous membranes, **characterized in that** a composition according to any one of Claims 1 and 3 to 7 is applied to the skin, the scalp, the hair, the nails and/or the mucous membranes.
